Europäisches Patentamt

European Patent Office

Office européen des brevets

(11) Publication number: **0 282 160 B1**

# EUROPEAN PATENT SPECIFICATION

(45) Date of publication of patent specification: **10.05.95**

(51) Int. Cl.⁶: **C12N 7/00**, C12Q 1/70, C12Q 1/04, G01N 33/569, C12Q 1/48

(21) Application number: **88301009.2**

(22) Date of filing: **08.02.88**

(54) **A method of obtaining a retrovirus-containing fraction from retrovirus-containing cells.**

(30) Priority: **07.02.87 GB 8702816**

(43) Date of publication of application:
**14.09.88 Bulletin 88/37**

(45) Publication of the grant of the patent:
**10.05.95 Bulletin 95/19**

(84) Designated Contracting States:
**AT BE CH DE ES FR GR IT LI LU NL SE**

(56) References cited:
**EP-A- 0 174 534**

**PROC. NATL. ACAD. SCI. USA, vol. 81, July 1984, pages 4188-4192; I. KEYDAR etal.: "Properties of retrovirus-like particles produced by a human breastcarcinoma cell line: Immunological relationship with mouse mammary tumor virusproteins"**

(73) Proprietor: **THE UNIVERSITY OF LIVERPOOL Mount Pleasant, Senate House, Abercromby Square, P.O. Box 147 Liverpool L69 3BX (GB)**

(72) Inventor: **Al-Sumidaie, Ayad Mohamed Khalaf 68 St. Ives Way Halewood Liverpool, L26 7YW (GB)**

(74) Representative: **Lyons, Andrew John et al ROYSTONS Tower Building Water Street Liverpool L3 1BA (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

CHEMICAL ABSTRACTS, vol. 93, no. 21, 24th
November 1980, pages 108-109,abstract no.
198232p, Columbus, Ohio, US; J. BLAKESLEE
et al.: "Induction ofretrovirus non-producer
human cells to producer cells by dex-
amethasone"

CHEMICAL ABSTRACTS, vol. 93, no. 1, 7th
July 1980, page 114, abstract no.1139j, Co-
lumbus, Ohio, US; B. GUILLEMAIN et al.: "In
vitro production andtitration assays of B-
tropic retroviruses isolated from C57BL
mouse tumorsinduced by radiation leukemia
virus (RadLV-Rs): effect of dexamethasone"

J. NATL. CANCER INST., vol. 60, no. 6, June
1978, pages 1305-1308; D.L. BRONSONet al.:
"Morphologic evidence for retrovirus pro-
duction by epithelial cellsderived from a hu-
man testicular tumor metastasis: Brief com-
munication"

CHEMICAL ABSTRACTS, vol. 107, no. 7, 17th
August 1987, page 204, abstract no.53214g,
Columbus, Ohio, US; M. ONO et al.: "Stimula-
tion of expression of thehuman endogenous
retrovirus genome by female steroid hor-
mones in human breastcancer cell line
T47D"

THE JOURNAL OF IMMUNOLOGY, vol. 131,
no. 5, November 1983, pages 2279-2281,
TheAmerican Association of Immunologists,
US; J.H. BERTOGLIO: "Monocyte-indepen-
dent stimulation of human B lymphocytes by
phorbol myristate acetate"

**Description**

The present invention relates to a method of obtaining a retrovirus-containing fraction from mononuclear cells containing a retrovirus. The use of such a method providing means for screening said cells for the presence of said retrovirus and also an isolated fraction enabling positive identification of the particular virus to be achieved.

It has long been suspected that cancer, and in particular, breast cancer may be a result of viral infection. In females the most common form of malignancy is carcinoma of the breast. This carcinoma is known to affect about 9 per cent of the adult female population and in females in the 40 to 54 age group is a major cause of death. A proble in diagnosis is that in many of these carcinomas the cancerous growth is slow, possibly taking up to 10 years for a 1 cm growth, and as a result of this slow growth, many carcinomas are not detected until the carcinoma is too advanced.

Whilst eary diagnosis of such cancers materially enhances the possibility of a cure it is evident that detection of the causal factor before the cancer becomes manifest would enable prevention measures to be developed. Thus providing a screening method to enable early and effective recognition of the presence of the causal factor would increase greatly the possibility of successfully treating many patients.

There is also no method of identifying an effective treatment for a patient with breast cancer. Different treatments may be tried and continued depending on the patient's response. But there is no blood test for monitoring the continuous effect of a treatment on the retrovirus present in mononuclear or monocytes from patients with breast cancer.

At present there is no method which can be used in screening for breast cancer in the very early stages and no method by which retroviruses are screened for directly, although it is known to screen for retroviruses indirectly by use of a mouse mammary tumor antibody, but because of the possibility of cross-reactivity that indirect method can give false positive results.

It is also known to screen for retroviruses by assaying for the enzyme reverse transcriptase. To do this the virus must undergo replication to provide enough reverse transcriptase to allow positive identification.

It is known that in genetically susceptible mice a virus, Murine mammary tumor virus, MMTV, can be the cause of breast cancers, the virus being detectable in the mammary tumor cells of the infected mice.

It is further known that virus particles of type C morphology can be enhanced in mammary tumor cells of mice by the use of adrenocorticoids. The use of these hormones results in increased production of virus related RNA-depended DNA polymerase, MMTV specific antigens, and B particles. This was shown when the synthetic glucocorticoid hormone, dexamethasone, was used in cultures of iododeoxyuridine (IUDR) stimulated mouse mammary tumour cells. In these experiments dexamethasone was added to the growth medium after IUDR treatment, IUDR being known to act as an inducer of leukemia and herpes viruses. (Fine et al. June 1974, Journal of the National Cancer Institute 52, 6, pages 1881-1884). Furthermore, in cell lines and primary explants derived from mammary tumours of several strains of mice the amount of virus production correlates with the level of virus-specific RNA. This suggests that in these cells, transcriptional controls are of primary importance in regulating the production of MMTV. Experiments with dexamethasone (a synthetic glucocorticoid) support this notion since cells treated with the hormone show parallel increases in virus production and intracellular virus-specific RNA. In contrast, a lymphoma cell line (S49) derived from a lymphoma induced by mineral oil in a BALB/c mouse contains large quantities of MMTV-specific RNA yet produces extremely low levels of virus. In these cells, mechanisms other than transcriptional controls may be important in regulating virus production. (Ringold 1975, Virology 65, pages 135-147).

However, MMTV differs in several respects from other members of the retrovirus family. For example it induces a high incidence of mammary adenocarcinomas as opposed to the more common leukemias and sarcomas associated with other retroviruses. (Dickson 1981, Journal of Virology January, 37 pages 36-47).

It is known that blood leukocytes including monocytes are attracted to diseased regions of the body in response to chemotactic agents. (Al-Sumidaie et al. 1984, Journal of Immunological Methods 75, pages 129-140). The leukocytes are believed to become involved in elimination and destruction of tumour cells. However it has surprisingly been shown that monocytes, from patients with breast carcinoma when incubated in vitro using an under agarose technique give rise to giant cell formation. These macrophage polykaryons are believed to be formed by fusion of macrophases derived from monocytes (Al-Sumidaie 1986, Jounral of Immunological Methods 91, pages 237-242).

It has been suggested that giant cell formation most likely results from virally mediated cell fusion. Possible explanations for the giant cell formation from monocytes are:-

(1) A defect in cellular immunity, resulting from viral infection of monocytes, may produce an increase in tumour incidence as a result of failure to eliminate abnormal cells arising by spontaneous mutation.

(2) All the cells in the body are infected by a virus which expresses itself in monocytes in the ability to form giant cells and in decreased migration and phagocytosis, but expresses itself in breast tissue as a carcinogenicis. (Al-Sumidaie et al. 1986, British Journal Surgery 73, pages 839-842).

A link can therefore be deduced between breast cancer and depressed monocyte functions in patients suffering from breast cancer. The question which has to be answered is whether a retrovirus could be shown to be that link.

It has now been surprisingly found that incubation of monocytes taken from patients with breast cancer, in a culture medium containing dexamethasone, Phorbol myristate acetate or $5^{'}$ azacytidine for 6 day caused release of particles in the supernatant which showed reverse transcriptase activity and hence indicated a retrovirus to be present. This result was all the more surprising when it is considered that no activity could be detected when a normal incubation period of 18 hours was used and the activity was very low when dexamethasone, Phorbol myristate acetate or $5^{'}$ azacytidine was omitted from the incubation mixture. This is unlike the case when adrenocorticoids were used to enhance MMTV detection in cultured murine carcinoma cells in which detection could be made within short incubation periods. Furthermore, when $5^{'}$ azacytidine or Phorbol myristate acetate was added to mouse mammary tumour cell cultures, reverse transcriptase activity doubled compared with the same cell line incubated in the presence of dexamethasone. Conversely, the reverse transcriptase activity released by mononuclear cells from patients with breast cancer was 3 to 7 times when these cells were incubated in the presence of $5^{'}$ azacytidine, while the activity increased 60 to 120 times when these cells were incubated in the presence of Phorbol myristate acetate compared with same samples incubated in the presence of dexamethasone.

In accordance with one embodiment of the present invention there is provided a method of forming a retrovirus-containing fraction from monocytes or mononuclear cells containing said virus comprising, preparing a suspension of separated monocytes or mononuclear cells in a culture medium, incubating said culture and separating the culture supernatant from said incubated culture, characterized in that an effective amount of glucocorticoid or of a leukemia or other viral or retroviral inducing drug is added to the suspension before and/or during the incubation of said cultures, and if desired separating a retrovirus-containing fraction from the separated supernatant.

In another embodiment the invention provides a method of detecting the presence of retroviruses in monocytes or mononuclear cells which is characterized by subjecting a culture of said cells to incubation in the presence of a glucocorticoid hormone or active derivative thereof or of a leukemia or other viral or retroviral inducing drug to form a retrovirus-containing fraction and subjecting the fraction to test procedures for detecting said virus.

In another embodiment the present invention provides a method of screening human beings suspected of having breast cancer for the presence of retrovirus characterized by subjecting a culture of monocytes or mononuclear cells, taken from the individual to be screened to incubation in a culture in the presence of an amount of a glucocorticoid or of a leukemia or other viral or retroviral inducing agent sufficient when retrovirus is present to give rise in the supernatant to particles containing said retrovirus and subjecting said particules when present to a test procedure which determines the presence of said virus.

In a further embodiment the present invention provides a method of converting a non-detectable form of retrovirus to a detectable form of said virus characterized by subjecting a specimen comprising monocytes or mononuclear cells containing a non-detectable form of said virus to incubation in a culture medium preferably for a period greater than 18 hours in the presence of a glucocorticoid or a leukemia or other viral or retroviral inducing agent to give rise to a fraction containing a detectable form of said virus.

The invention also provides a vehicle for effecting said conversion from a non-detectable form of retrovirus to a detectable form which comprises a culture medium containing an effective amount of glucocorticoid hormone or an active derivative thereof or of a leukemia or other viral or retroviral inducing agent.

The glucocorticoid hormone may be, for example, in the form of the synthetic drug dexamethasone and the leukemia or viral inducing agent may be, for example, tetradecanoyl phorbol acetate, especially Phorbol myristate acetate (TPA), azacytidine, especially $5^{'}$ azacytidine, aminopeterin, 8-azaguanine, azaserine, tuftsin, 2-amino-6-mercaptopurine, carboxyethyl-gama-aminobutyric acid, demecolcine, dimethyl sulfoxide, ouabain, polyethyleneglycol, pristane or other viral or retroviral stimulators, preferably for periods of 3 to 60 days. By way of example only, more specific embodiments of the present invention will now be described:

In accordance with one more specific embodiment of the present invention there is provided a method of forming a retrovirus-containing fraction from monocytes or mononuclear cells or preferably but not essentially purified monocytes containing said virus comprising preparing a suspension of separated mononuclear cells or purified monocytes in a culture medium, incubating said culture and separating the culture, characterized in that an effective amount of dexamethasone, Phorbol myristate acetate or $5^{'}$

azacytidine is added to the suspension before and/or during the incubation of said cultures, and if desired separating a retrovirus-containing fraction from the separated supernatant. In a preferred embodiment, said culture medium comprises Eagle's medium supplemented with 10 per cent foetal calf serum, said incubation time is 3 to 30 days, preferably 6 days and said effective amount of Phorbol myristate acetate is 330 ng per ml of incubating culture medium. The incubation is preferably carried out at substantially 37 degrees C and in an atmosphere of 5 per cent $CO_2$ in air. Said retrovirus-containing fraction is separated preferably though not necessarily by filtration means, filtration preferably being carried out with a 220 nm filter. Said filtrate is centrifuged at high speed, preferably at 10,000 G for 5 to 15 minutes, and maintaining a temperature preferably of 18 degrees C and the pellet being suspended in a suitable medium as required.

In another more specific embodiment the invention provides a method of detecting the presence of retroviruses in monocytes said method comprising incubating said culture cells in the presence of Phorbol myristate acetate (330 ng per ml of incubating medium, $10^{-6}$ M dexamethasone or 15 $\mu$M of 5$'$ azacytidine as hereinbefore defined, said retrovirus-containing fraction being subjected to detection means. Said detection means preferably comprises a reverse transcriptase assay. For said assay the resuspended high-speed pellet obtained from the supernatant is disrupted by the addition of a non-ionic detergent. Preferably NP40 (final concentration 0.2% v/v) and dithiothreitol (DTT) (final concentration 50mM) and incubated under suitable conditions, preferably at 20 degrees C for 15 minutes. The reverse transcriptase activity is then measured by a standard assay procedure using the divalent ion $Mg2+$ due to its preferential effect with the human cells. As an alternative an assay procedure using $Mn2+$ may also be used. Thus, said reverse transcriptase activity is assayed by measuring the incorporation of radioactively labelled deoxycytidine triphosphate (dCTP) into acid-precipitable material, dependent on the presence of a synthetic RNA template. The reaction mix contains a final volume of 100 $\mu$l, 45 $\mu$l of extract, 5 $\mu$mol "tris"-HC1 pH 8.3, 5 $\mu$mol KC1, 2.5 $\mu$mol DDT, 0.6 $\mu$mol MC1$_2$ , 0.16 $\mu$mol each deoxyadenosine triphosphate (dATP), deoxythymidine triphosphate (dTTP), deoxyguanosine triphosphate (dGTP), 0.05 $\mu$mol dCTP, 5 $\mu$Ci (alpha $^{32}$ p) dCTP (3000 Ci/mmol), 0.5 $\mu$g oligodeoxycytidylic acid (oligo d(p$^c$)$_8$), 0.5. $\mu$g polyguanylic acid. The reaction is incubated at 37 degrees C for 1 hour. The reaction is stopped by the addition of 0.4 ml of 10 percent (w/v) trichloroacetic acid (TCA) and 25 Mg of calf thymus DNA. The DNA was precipitated overnight at - 20 degrees C. The DNA was sonicated at periods between 5 to 1 minute preferably 30 seconds. The precipitated radioactive DNA is collected by filtration onto a GF/C glass-fibre filter, and washed with 30 ml of 5 percent (w/v) TCA, the radioactivity on the filter was measured by means of a scintillation counter.

Whilst detection has been described by the means of the aforegoing example, alternative detection means, such as negative staining electron microscopy of the pellet present in the supernatant of incubated monocytes or mononuclear cells; electron microscopic examination of incubated monocytes or mononuclear cells; histoimmunoassay; immunocytochemical assay, particularly immunogold or immunosilver staining-of incubated or fresh monocytes or mononuclear cells; giant cell formation by monocytes or antigen antibody reaction, for example, peroxidase antiperoxidase, alkaline phosphatase- antialkaline phosphatase, Avidin-biotin or immunosorbent assays (ELISA). The antibody could be raised as polyclonal or monoclonal using, for example, rabbits, horses, goats, sheep, swine or mice. Alternatively, the method of the invention could be used for cloning and developing probes for the virus.

In yet another more specific embodiment of the present invention, there is provided a method of screening human beings suspected of having breast cancer for the presence of retrovirus characterized by subjecting said culture of monocytes taken from said individual to be screened as hereinbefore defined and subjecting said centrifuged filtrate to a screening means to determine the presence of said retrovirus.

In a further more specific embodiment the present invention provides a method of converting a non-detectable form of retrovirus to a detectable form of said virus by subjecting monocytes containing a non-detectable form of said virus to incubation in a culture medium, preferably for more than 18 hours, containing Phorbol myristate acetate, preferably at a concentration of 330 ng per ml of incubating culture medium, dexamethasone, preferably at a concentration up to $10^{-6}$ M, or 5$'$ azacytidine, preferably at a concentration of 15 $\mu$M, to give rise to a fraction containing a detectable form of said virus.

In another embodiment the invention provides a vehicle for effecting said conversion from a non-detectable form of retrovirus to a detectable form which comprises a culture medium containing Phorbol myristate acetate preferably at a concentration of 330 ng per ml incubating culture medium, dexamethasone, preferably at a concentration of $10^{-6}$ M of 5$'$ azacytidine, preferably at a concentration of 15 $\mu$M.

By way of a more specific embodiment of the present invention the blood collected for monocytes preparation can be treated with sodium or lithium heparin without interferring with the method for preparing mononuclear cells. The blood can further be kept, preferably in polycarbonate tubes, for a period of up to 8 hours at around 4 degrees C before undergoing mononuclear cell separation.

The mononuclear cells can be further stored at around 4 degrees C for up to 24 hours when in a suitable buffered solution.

Furthermore said culture medium for incubation of said monocytes or mononuclear cells can be prepared with or without antibiotics. Said media containing a suitable feed in particular a good protein source, preferably foetal calf serum at a concentration of between 5 and 20 preferably 7.5 to 12.5 per cent more preferably 10 per cent. The pH of said medium being kept within the range pH 6.8 to 8 preferably to 7.6 especially 7.4. Said culture medium being supplied with air containing $CO_2$ at a concentration of 4-8 percent preferably 5 percent. Said culture being maintained at a temperature of between 34 and 40 preferably between 36 and 40, especially 37.3 degrees C. The Phorbol myristate acetate, can be added to said medium at any period during the incubation procedure but must be added at least 2 days before the reverse transcriptase assay for best results. The period of incubation being maintained for up to 30 days preferably 6 days. Similar factors apply when 5' azacytidine or dexamethasone are used.

The preferred concentrations for use of said chemicals being in the range 100 to 600 ng per ml of culture medium preferably 330 ng for Phorbol myristate acetate and $10^{-5}$ M to $10^{-7}$ M preferably $10^{-6}$ M for dexamethasone and in the range 5 $\mu$M to 50 $\mu$M, preferably 10 $\mu$M to 20 $\mu$M, especially 15 $\mu$M for 5' azacytidine.

The monocytes or mononuclear cells can be separated from the said medium by a slow speed centrifuge in the range 600 to 800 xg for 10 to 20 minutes at a temperature of between 4 degrees and 20 degrees C. The monocytes or the mononuclear cells can then be resuspended in fresh medium and procedures repeated as necessary.

In accordance with a more specific embodiment of forming said retrovirus containing supernatant, said supernatant is separated from the monocytes or the mononuclear cells by either filtration through a suitable membrane with a pore size which allows the virus to pass through, yet retains the monocytes or the mononuclear cells, said filter preferably being 220 nm or by centrifugation at 600-800 xg for 10 to 20 minutes.

Said supernatant containing said viral particles may then undergo a high-speed centrifugation to precipitate said viral particles, said centrifugation being carried out at 8,000 to 120,000 xg preferably 12,000 xg at 4 degrees C to 20 degrees C for 5 to 120 minutes, preferably for 12 minutes.

In another more specific embodiment the detection means provide by assaying reverse transcriptase is dependent upon the viability of the monocytes or the mononuclear cells, which can be determined on an aliquot of the sample after incubation with the Phorbol myristate acetate, dexamethasone or 5' azacytidine.

Furthermore, the detection means proved equally effective in determing retrovirus activity in either male or female subjects with breast cancer.

The present invention also provides in a specific embodiment a method of detecting the effect of surgery, such as mastectomy with or without lymph node clearance, Halsted radical mastectomy, modified radical mastectomy, quadrantectomy or lumpectomy with or without lymph node clearance on the retrovirus present in the mononuclear cells or monocytes from an individual having breast cancer.

The method of the invention may be used as a tool for selecting an effective treatment or treatments for a breast cancer before actually administering that treatment or treatments. The method of the invention may also be used to monitor the effectiveness of treatment or treatments on a patient already undergoing such treatment or treatments. These treatments may be single or combined and may include the following:

(1) Surgical treatment, such as Halsted radical mastectomy, modified radical mastectomy, quadrantectomy or lumpectomy, optionally with radiotherapy, Cytotoxic drugs, hormonal therapy, viral or retroviral inhibitors or a combination of all.

(2) Radiotherapy, and in particular, though not exclusively, radiotherapy in locally advanced breast cancer or radiotherapy in metastatic breast cancer, or optionally with cytotoxic drugs, hormonal therapy or viral or retroviral inhibitors.

(3) Cytotoxic drugs (single or combined), for example L-phenylalanine mustards, 5-fluorouracil, adriamylin, Cyclophosphamide, methotrexate, vincristine and epirubicin, optionally with hormonal therapy or viral or retroviral inhibitors.

(4) hormonal therapy, such as with tamoxifen or a derivative thereof, progesterone, progestines,

(4) hormonal therapy, such as with tamoxifen or a derivative thereof, progesterone, progestines, medroxyprogesterone, norethisterone, megestrol, androgens, aminoglutethimide, oestrogen, cortecosteroids, prolactin or antiprolactin agents, optionally with viral or retroviral inhibitors.

(5) viral or retroviral inhibitors, for example interferons, lymphokines, azidothymidine, N-methylisatin-beta-4': 4-diethylthiosemicarbazone, 3'-azido, 3'-amino, 2', 3'-unsaturated, and 2', 3'-dideoxy analogues of pyrimidine deoxyribonucleosides, 2-deoxyglucose, tunicamycin or their derivatives, phosphonoformic acid, carbonyl diphosphate optionally with other forms of treatments as above in 1 to 4.

Another use of the invention may be to detect the effect of drugs such as viral, antiviral, hormonal, and cytotoxic drugs for treatment of or prophylaxis for carcinomas by incubating such drug with the monocytes or mononuclear cells suspended in a suitable medium during culture of or during the assay for the retrovirus present in these cells, for example, by incubating the monocytes or mononuclear cells in the presence of the viral or retroviral stimulator.

The embodiments described hereinbefore are further illustrated by the following examples.

PREPARATIVE EXAMPLES

Preparative Example 1

Blood Collection

Peripheral venous blood (40 ml) was collected from each subject in plastics tubes containing 10 IU preservative free heparin per ml. The blood was taken from the patients when they first presented in clinic with breast lumps. Full biochemical (sequential multichannel analyser with computer, SMAC) and haematological analysis revealed no abnormalities in either patients or controls.

Mononuclear Cell Separation and Monocyte Purification:

Mononuclear cells were separated from blood by centrifugation over Ficoll-Hypaque, 1.077 gm/ml density (Boyum 1968, Scandinavian Journal of Clinical and Laboratory Investigation 21 (suppl. 97), pages 77-89) and washed 3 times with ice cold buffered salt solution (BSS) prepared from 8.0 gm $NaC1$, 0.2 g KCl, 1.15g $Na_2 HPO_4$, 0.2 g $KH_2 PO_4$ and 0.2 g glucose in 1 litre sterile distilled water.

Monocytes were purified on a discontinuous density gradient of Percoll. Stock Percoll was prepared by mixing 9 parts of Percoll density 1.13 g/ml with 1 part of 10 times strength Eagle's medium. The 3 densities of Percoll were prepared as shown in Table I. Up to $40 \times 10^6$ mononuclear cells were suspended in 2 ml of Percoll density 1.074 g/ml and placed in a 10 ml polycarbonate centrifuge tube (Nunc, Denmark). Two millilitres of Percoll density 1.066 g/ml were gently layered over the first layer and another 2 ml of Percoll density 1.057 g/ml gently layered on top of the latter. The tube was centrifuged at 2200 xg for 90 minutes at room temperature. After centrifugation, 3 bands of cells could be identified. The monocyte rich band was found at the interface of the 1.057 g/ml and the 1.066 g/ml density gradient of Percoll. This band was carefully removed and washed 3 times with ice cold BSS, and a sample of cells taken for non-specific esterase staining, total and viability counts. The total number of cells was adjusted to $25 \times 10^6$ cells/ml in Eagle's medium pH 7.1.

Cells were counted in a haemocytometer, and viability determined by trypan blue dye exclusion. Non-specific esterase staining used was by the method Yam et al., 1971 American Journal of Pathology, 55 pages 283-290.

All cell manipulations were carried out using sterile materials and solutions in a laminar flow hood.

## TABLE I

## PREPARATION OF THE THREE ISO-OSOMOLAR (osM = 310) mosmol/1) DENSITIES OF PERCOLL USED IN THE DISCONTINUOUS DENSITY GRADIENT

|  | Stock Solution (ml) | Eagle's medium (ml) |
|---|---|---|
| Percoll s.g. 1.057 g/ml | 42.4 | 57.6 |
| Percoll s.g. 1/066 g/ml | 50.00 | 50.00 |
| Percoll s.g. 1.074 g/ml | 56.65 | 43.35 |

## PREPARATION OF CELL-FREE CULTURE MEDIUM

The monocytes from patients and controls were suspended in Eagle's medium supplemented with 10 percent foetal calf serum and Phorbol myristate acetate (300 ng per ml of culture medium). After 6 days incubation at 37 degrees C in 5 percent $CO_2$ in air, in a humidified incubator, the supernatant was filtered using a 220 nm filter. The filtrates were centrifuged at 12,000 xg for 12 minutes at 18 degrees C. For reverse transcriptase assay the pellets were suspended in 1 ml of T.N.E. medium (10 mH "tris"-HC1 pH 8.3, 150 nM NaC1, 2 nM EDTA). For stimulation of giant cell formation among control monocytes or for electron microscopy the pellets were suspended in Eagle's medium (1 ml). For negative-staining electron microscopy the suspension was fixed with 0.25 percent glutaraldehyde-cacodylate, mixed with phosphotungstic acid (2 percent) and examined using a Philips 301 electron microscope.

## REVERSE TRANSCRIPTASE ASSAY:

The resuspended high speed pellets obtained from the cell-free culture medium of incubated mononuclear cells or monocytes or from mouse mammary tumour cells were disrupted by the addition of the nonionic detergent NP40 (final concentration 0.2 percent v/v) and dithiothreitol (final concentration 50 nM) and incubated at 20 degrees C for 15 minutes.

Reverse transcriptase activity was assayed by measuring the incorporation of radioactively labelled dCTP into acid-precipitable material, dependent on the presence of a synthetic RNA template. The reaction mix contained, in a final volume of 100 $\mu$l, 45 $\mu$l of extract, 5 $\mu$mol "tris"-HCl pH 8.3, 5 $\mu$mol KCl, 2.5 $\mu$mol DTT, 0.6 $\mu$mol $MgCl_2$ , 0.16 $\mu$mol each dATP, dTTP, dGTP, 0.05 $\mu$mol dCTP, 5 $\mu$Ci (alpha[32] p) dCTP (3000 Ci/mmol), 0.5 $\mu$g oligodeoxycytidylic acid (oligo d(p$^c$)$_8$), 0.5 $\mu$g polyguanylic acid. The reaction was incubated at 37 degrees C for 1 hour. The reaction was stopped by the addition of 0.4 ml of 10 percent (w/v) trichoracetic acid (TCA) and 25 $\mu$g of calf thymus DNA. The DNA was precipitated overnight at -20 degrees C. The precipitated radioactivity was collected by filtration onto a GF/C glass-fibre filter and washed with 30 ml of 5 percent (w/v) TCA. The radioactivity on the filter was measured by scintillation counting.

All assays were performed in triplicate.

Preparative Example 2

In this, the procedure is as for preparative Example 1 except that in the preparation of the cell-free culture medium in which monocytes or mononuclear cells had been incubated there is the following difference.

The monocytes or mononuclear cells from patients and controls were suspended in Eagle's medium supplemented with 10 percent foetal celf serum and Phorbol myristate acetate at a concentration of 330 ng per ml of culture medium or 5$'$ azacytidine at 15 $\mu$M. After 6 days incubation at 37 degrees C in 5% $CO_2$ in air, in a humidified incubator, the supernatant was filtered using a 200 nm filter. The filtrates were centrifuged at 12,000 xg for 12 minutes at 18 degrees C. For reverse transcriptase assay the pellets were suspended in 1 ml of T.N.E. medium (10 nM "tris"-HCl pH 8.3, 150 nM NACl, 2 mM EDTA). For stimulation of giant cell formation among control monocytes or for electron microscopy the pellets were suspended in Eagle's medium (1 ml). For negative-staining electron microscopy the suspension was fixed with glutaraldehyde-cacodylate, mixed with phosphotungstic acid (2 percent) and examined using a Philips 301 electron microscope.

SUCROSE GRADIENT

A high speed pellet was prepared from the cell-free culture medium of incubated monocytes from patients with breast cancer as described previously. The pellets were resuspended in 100 $\mu$l of T.N.E. medium and layered on discontinuous densities of sucrose 20, 30, 40 and 60 percent in T.N.E. gradient and centrifuged (preferably but not necessarily in Beckman SW65 rotor) at 120,000 xg for 16 hours at 4 degrees C. Fractions (250 ml) were collected by piercing the bottom of the tube, diluted in T.N.E. centrifuged at 12,000 xg for 12 minutes and the pellets were assayed for reverse transcriptase. The density of the sucrose was determined using a refractometer.

Example 3 shows the reverse transcriptase activity in the cell-free culture medium in which monocytes or mononuclear cells from patients with breast cancer had been indubated was associated with a particle having a buoyant density between 1.165 and 1.18 g/ml on a sucrose density gradient.

e.g. 3

| Fraction number | g/ml sucrose density | Reverse transcriptase activity (cpm) |
|---|---|---|
| 1 | 1.29 | 20,000 |
| 2 | 1.28 | 1,000 |
| 3 | 1.26 | 2,000 |
| 4 | 1.23 | 500 |
| 5 | 1.21 | 2,000 |
| 6 | 1.19 | 500 |
| 7 | 1.18 | 40,000 |
| 8 | 1.165 | 37,000 |
| 9 | 1.155 | 4,000 |
| 10 | 1.145 | 4,500 |
| 11 | 1.13 | 200 |
| 12 | 1.12 | 300 |
| 13 | 1.11 | 300 |
| 14 | 1.10 | 600 |
| 15 | 1.10 | 40 |

Example 4 shows the results obtained using the preparative method 1 hereinbefore described in which the chemical dexamethasone was used in the culture medium containing monocytes or mononuclear cells. In which the monocytes or the mononuclear cells were obtained from breast cancer patients and age matched controls respectively.

| | No of subjects tested using preparation example 1 | Reverse Transcriptase or retrovirus containing fraction | Giant Cell formation | Examination for viral particles using Electron Microscope |
|---|---|---|---|---|
| e.g. 4 isolated fraction from cultured monocytes of breast cancer patients | 28 | 27+'ve | 28+'ve | 28+'ve |
| isolated fraction from cultured monocytes or mononuclear cells of age matched controls | 18 | 2+'ve | 2+'ve | 2+'ve |

Example 5 shows the importance of dexamethasone on the reverse transcriptase activity in a patient with breast cancer.

| Sample | Reverse transcriptase activity |
|---|---|
| Isolated fraction prepared from monocytes or mononuclear cells of a breast cancer patient treated with dexamethasone | 2770 cpm (6.1 picomoles dCTP incorporated) |
| Isolated fraction prepared from monocytes, of a breast cancer patient, not treated with dexamethasone | 620 cpm (1.4 picomoles dCTP incorporated) |

Example 6 shows comparisons of reverse transcriptase activity on isolated fractions, of age matched controls, treated with and without dexamethasone respectively.

12

e.g. 6

| Sample | Reverse transcriptase Activity |
|---|---|
| Isolated fraction of control subject, with dexamethasone | 228 cpm (0.5 picomoles dCTP incorporated) |
| Isolated fraction of control subject, without dexamethasone | 135 cpm (0.3 picomoles dCTP incorporated) |

Example 7 shows the effect of dexamethasone in cultures of monocytes or mononuclear cells, 5' azacytidine in cultures of monocytes or mononuclear cells and Phorbol myristate acetate in cultures of monocytes or mononuclear cells in terms of reverse transcriptase activity in a patient with breast cancer using $Mg^{+2}$ in the assay.

| | R.T. Activity |
|---|---|
| Phorbol myristate acetate treated isolated fraction | 112761 cpm (248 picomoles dCTP incorporated) |
| Azacytidine treated isolated fraction | 7718 cpm (17 picomoles dCTP incorporated) |
| Dexamethasone treated isolated fraction | 2410 cpm (5.3 picomoles dCTP incorporated) |

```
Isolated fraction from        510 cpm

culture containing no         (1.1 picomoles dCTP

dexamethasone, 5'             incorporated)

azacytidine, or

Phorbol myristate

acetate
```

Example 8 shows the effect of $Mn^{+2}$ or $Mg^{2+}$ when used in the reverse transcriptase assay with a patient with a breast cancer using Phorbol myristate acetate as retroviral stimulator.

```
Sample                        R.T. Activity


Mg 2+                 =        42710 cpm

                              (94 picomoles dCTP

                              incorporated)



Mn 2+                 =        18260 cpm

                              (40 picomoles dCTP

                              incorporated)
```

Example 9 shows the effect of the period of incubation one reverse transcriptase activity in the isolated fraction from a patient with breast cancer and a control subject using Phorbol myristate acetate as viral stimulator, and $Mg^{2+}$ in the assay.

|  |  | Time | R.T. Activity |
|---|---|---|---|
| 1. | Breast cancer patient |  |  |
|  | at | 8 days | 32590 cpm (71.6 picomoles dCTP incorporated |
|  | at | 15 days | 35830 cpm (78.7 picomoles dCTP incorporated) |
| 2. | Control subjects |  |  |
|  | at | 10 days | 250 cpm (0.5 picomoles dCTP incorporated) |
|  | at | 16 days | 310 cpm (0.7 picomoles dCTP incorporated) |

Example 10 shows the effect of the time of centrifugation of the cell-free culture medium in which monocytes or mononuclear cells were incubated on reverse transcriptase activity in a patient with breast cancer.

15

| Time | R.T. Activity |
|------|---------------|
| 5 minutes | 55480 cpm (122 picomoles dCTP incorporated) |
| 10 minutes | 71470 cpm (157 picomoles dCTP incorporated) |
| 12 minutes | 75460 cpm (166 picomoles dCTP incorporated) |
| 15 minutes | 75950 cpm (167 picomoles dCTP incorporated) |
| 60 minutes | 77010 cpm (169 picomoles dCTP incorporated) |

Example 11 shows the effect of mastectomy on reverse transcriptase activity in patients with breast cancer.

Reverse transcriptase activity is expressed in cpm per 45 $\mu$l of sample and picomoles of dCTP incorporated per 45 $\mu$l of sample.

| Patients | before operation | 3 months after operation |
|---|---|---|
| 1 | 50927 cpm (134 picomoles) | 54712 cpm (120 picomoles) |
| 2 | 32616 cpm ( 72 picomoles) | 31792 cpm ( 70 picomoles) |
| 3 | 50148 cpm (110 picomoles) | 52744 cpm (116 picomoles) |
| 4 | 27547 cpm ( 61 picomoles) | 24080 cpm ( 53 picomoles) |
| 5 | 47175 cpm (104 picomoles) | 51684 cpm (114 picomoles) |
| 6 | 21774 cpm ( 48 picomoles) | 19524 cpm ( 43 picomoles) |

Example 12 shows the effect of mastectomy with lymph node clearance on reverse transcriptase activity in patients with breast cancer. Reverse transcriptase is expressed in cpm per 45 $\mu$l of sample and picomoles of dCTP incorporated per 45 $\mu$l of sample.

| Patients | before operation | 3 months after operation |
|---|---|---|
| 1 | 27154 cpm (60 picomoles) | 25517 cpm (56 picomoles) |
| 2 | 18178 cpm (40 picomoles) | 16211 cpm (36 picomoles) |
| 3 | 40114 cpm (88 picomoles) | 42898 cpm (94 picomoles) |
| 4 | 25296 cpm (56 picomoles) | 22686 cpm (50 picomoles) |

Example 13 shows the effect of lumpectomy with lymph node clearance followed by a course of radiotherapy to the chest wall and the axillary region. Reverse transcriptase activity is expressed as cpm per 45 $\mu$l of sample or picomoles of dCTP incorporated per 45 $\mu$l of sample.

| Patients | before treatment | 3 months after treatment |
|---|---|---|
| 1 | 22862 cpm | 10195 cpm |
| | (50 picomoles) | (22 picomoles) |
| 2 | 16932 cpm | 7912 cpm |
| | (37 picomoles) | (17 picomoles) |
| 3 | 45660 cpm | 38976 cpm |
| | (100 picomoles) | (86 picomoles) |
| 4 | 35734 cpm | 9788 |
| | (79 picomoles) | (22 picomoles) |

Example 14 shows the effect of tamoxifen (taken by patients with breast cancer) on reverse transcriptase activity. Reverse transcriptase activity is expressed as cpm per 45 $\mu$l of sample or picomoles of dCTP incorporated per 45 $\mu$l of sample.

| Patients | before treatment | 3 months after treatment |
|---|---|---|
| 1 | 50799 cpm | 2457 cpm |
| | (112 picomoles) | ( 5 picomoles) |
| 2 | 18162 cpm | 3146 cpm |
| | ( 40 picomoles) | ( 7 picomoles) |
| 3 | 27005 cpm | 23125 cpm |
| | ( 59 picomoles) | (51 picomoles) |
| 4 | 22972 cpm | 4492 cpm |
| | ( 50 picomoles) | (10 picomoles) |

Example 15 shows the effect of different concentrations of tamoxifen on reverse transcriptase activity. The drug was added during the incubation of monocytes or mononuclear cells from a patient with breast cancer. Reverse transcriptase activity is expressed in cpm 45 $\mu$l of sample and picomoles of dCTP

18

incorporated per 45 μl of sample.

|  | Reverse transcriptase activity |
|---|---|
| No tamoxifen | 32635 cpm (72 picomoles) |
| 150 ng per ml tamoxifen | 21669 cpm (48 picomoles) |
| 300 ng per ml tamoxifen | 7895 cpm (17 picomoles) |
| 600 ng per ml tamoxifen | 743 cpm (1.6 picomoles) |

Example 16 shows the effect of different concentrations of medroxyprogesterone acetate on reverse transcriptase activity. The drug was added during the incubation of monocytes or mononuclear cells from a patient with breast cancer. Reverse transcriptase activity is expressed in cpm per 45 μl of sample and picomoles of dCTP incorporated per 45 μl of sample.

|  | Reverse transcriptase activity |
|---|---|
| No medroxyprogesterone | 25016 cpm (55 picomoles) |
| 100 ng per ml medroxyprogesterone | 12995 cpm (29 picomoles) |
| 200 ng per ml medroxyprogesterone | 2479 cpm ( 5 picomoles) |
| 400 ng per ml medroxyprogesterone | 1078 cpm ( 2 picomoles) |

Example 17 shows the effect of 5-fluorouracil on reverse transcriptase activity. The drug was added during the incubation of monocytes or mononuclear cells from a patient with breast cancer. Reverse transcriptase activity is expressed as cmp per 45 μl of sample and picomoles of dCTP incorporated per 45 μl of sample.

|                        | Reverse transcriptase |
| ---------------------- | --------------------- |
|                        | activity              |
| No drug                | 22010 cpm             |
|                        | (48 picomoles)        |
| with 5-fluorouracil    | 3408 cpm              |
|                        | ( 7 picomoles)        |

Example 18 shows the effect of aminoglutethimide on reverse transcriptase activity. The drug was added during their incubation of monocytes or mononuclear cells from a patient with breast cancer. Reverse transcriptase activity is expressed in cpm per 45 $\mu$l of sample and picmoles of dCTP incorporated per 45 $\mu$l of sample.

|                        | Reverse transcriptase |
| ---------------------- | --------------------- |
|                        | activity              |
| No drug                | 32715 cpm             |
|                        | (72 picomoles)        |
| with aminoglutethimide | 5423 cpm              |
|                        | (12 picomoles)        |

Example 19 shows the effect of tamoxifen on reverse transcriptase activity. The drug was added to the incubated monocytes or mononuclear cells from patients with breast cancer. Reverse transcriptase activity is expressed in cpm per 45 $\mu$l of sample and picomoles of dCTP incorporated per 45 $\mu$l of sample.

| Sample | without tamoxifen | with tamoxifen |
| ------ | ----------------- | -------------- |
| 1      | 44546 cpm         | 4220 cpm       |

|   | (98 picomoles) | ( 9 picomoles) |
|---|---|---|
| 2 | 25792 cpm | 6217 cpm |
|   | (57 picomoles) | (14 picomoles) |
| 3 | 38117 cpm | 835 cpm |
|   | (84 picomoles) | (1.8 picomoles) |
| 4 | 20170 cpm | 18189 cpm |
|   | (44 picomoles) | (40 picomoles) |

Example 20 shows the effect of tamoxifen on reverse transcriptase activity. The drug was added during the reverse transcriptase assay to the pellet from the supernatant of incubated monocytes or mononuclear cells from a patient with breast cancer. Reverse transcriptase is expressed in cpm per 45 $\mu$l of sample and picomoles of dCTP incorporated per 45 $\mu$l of sample.

|   | Reverse transcriptase activity |
|---|---|
| without tamoxifen | 47332 cpm |
|   | (104 picomoles) |
| with tamoxifen | 489 cpm |
|   | (1.1. picomoles) |

**Claims**

1. A method of forming a retrovirus-containing fraction from human monocytes or mononuclear cells containing said virus comprising, preparing a suspension of separated monocytes or mononuclear cells from a human having, or suspected of having breast cancer in a culture medium, incubating said culture and separating the culture supernatant from said incubated culture, wherein an effective amount of a glucocorticoid or of a leukemia or viral inducing drug is added to the suspension before and/or during the incubation of said cultures.

2. A method as claimed in claim 1 comprising the step of separating a retrovirus containing fraction from the separated supernatant.

3. A method as claimed in claim 1, wherein the glucocorticoid hormone is in the form of dexamethasone.

4. A method as claimed in claim 1, wherein the leukemia or viral inducing agent is phorbol myristate acetate or 5' azacytidine.

5. A method as claimed in claim 4, wherein the culture medium comprises Eagle's medium supplemented with 10% foetal calf serum, said incubation time is 3 to 30 days and said effective amount of phorbol myristate acetate is 330 ng per ml of incubating culture medium.

6. A method as claimed in claim 5, wherein the incubation is carried out at substantially 37 degrees C and in an atmosphere of 5% $CO_2$ in air.

7. A method of detecting the presence of retroviruses in human monocytes or mononuclear cells from a human having, or suspected of having breast cancer comprising subjecting a culture of said cells to incubation in the presence of a glucocorticoid hormone or active derivative thereof or of a leukemia or other viral inducing drug to form a retrovirus containing fraction and subjecting the fraction to test procedures for detecting said virus.

8. A method as claimed in claim 7 comprising incubating said culture cells in the presence of phorbal myristate acetate (330 ng per ml of incubating medium) $10^{-6}$ M dexamethasone or 15 $\mu$M of 5' azacytidine, said retrovirus containing fraction being subjected to detection means.

9. A method as claimed in claim 8, wherein said detection means comprises a reverse transcriptase assay.

10. A method as claimed in claim 8, wherein the detection means comprises negative staining electron microscopy.

11. A method as claimed in claim 8, wherein the detection means comprises giant cell formation.

12. A method as claimed in claim 8, wherein the detection means comprises antibody reaction.

13. A method of screening human beings suspected of having breast cancer for the presence of retrovirus as comprising subjecting a culture of monocytes or mononuclear cells, taken from the individual to be screened, to incubation in a culture in the presence of an amount of a glucocorticoid or of a leukemia or other viral inducing agent sufficient when retrovirus is present to give rise in the supernatant to particles containing said retrovirus and subjecting said particles when present to a test procedure which determines the presence of said virus.

14. A method as claimed in claim 13 comprising subjecting said culture of monocytes taken from said individual to be screened and subjecting said centrifuged filtrate to a screening means to determine the presence of said retrovirus.

**Patentansprüche**

1. Verfahren zur Herstellung einer Retrovirus-haltigen Fraktion aus humanen Monozyten oder mononukleären Zellen, die das Virus enthalten, umfassend die Herstellung einer Suspension in einem Kulturmedium von isolierten Monozyten oder mononukleären Zellen aus einem Menschen mit Brustkrebs oder beim dem der Verdacht auf Brustkrebs besteht, die Inkubation der Kultur und die Abtrennung des Kulturüberstandes aus der inkubierten Kultur, wobei eine wirksame Menge eines Glucocorticoids oder eines Leukämie- oder Virus-induzierenden Wirkstoffs vor und/oder während der Inkubation der Kulturen zu der Suspension gegeben wird.

2. Verfahren gemäß Anspruch 1, umfassend den Schritt der Abtrennung einer Retrovirus-haltigen Fraktion aus dem abgetrennten Überstand.

3. Verfahren gemäß Anspruch 1, wobei das Glucocorticoid-Hormon in Form von Dexamethason vorliegt.

4. Verfahren gemäß Anspruch 1, wobei das Leukämie- oder Virusinduzierende Agens Phorbolmyristatacetat oder 5'-Azacytidin ist.

5. Verfahren gemäß Anspruch 4, wobei das Kulturmedium mit 10% fötalem Kälberserum ergänztes Eagle's-Medium umfaßt, wobei die Inkubationsdauer 3 bis 30 Tage beträgt, und wobei die wirksame Menge an Phorbolmyristatacetat 330 ng/ml des inkubierten Kulturmediums beträgt.

6. Verfahren gemäß Anspruch 5, wobei die Inkubation im wesentlichen bei 37°C und in einer Atmosphäre von 5% $CO_2$ in Luft durchgeführt wird.

**7.** Verfahren zur Detektion der Gegenwart von Retroviren in humanen Monozyten oder mononukleären Zellen aus einem Menschen mit Brustkrebs, oder bei dem der Verdacht auf Brustkrebs besteht, wobei man eine Kultur der Zellen einer Inkubation in Gegenwart eines Glucocorticoid-Hormons oder eines aktiven Derivates davon oder eines Leukämie- oder eines ein anderes Virus induzierenden Wirkstoffes unterzieht, wodurch eine Retrovirus-haltige Fraktion gebildet wird, und wobei man die Fraktion Untersuchungsverfahren zur Detektion des Virus unterzieht.

**8.** Verfahren gemäß Anspruch 7, umfassend die Inkubation der Kulturzellen in Gegenwart von Phorbolmyristatacetat (330 ng/ml des Inkubationsmediums), $10^{-6}$ M Dexamethason oder 15$\mu$M 5'-Azacytidin, wobei die Retrovirus-haltige Fraktion Detektionsmaßnahmen ausgesetzt wird.

**9.** Verfahren gemäß Anspruch 8, wobei die Detektionsmaßnahme einen Reverse Transcriptase-Assay umfaßt.

**10.** Verfahren gemäß Anspruch 8, wobei die Detektionsmaßnahme eine Negativ-kontrast-Elektronenmikroskopie umfaßt.

**11.** Verfahren gemäß Anspruch 8, wobei die Detektionsmaßnahme die Bildung von Riesenzellen umfaßt.

**12.** Verfahren gemäß Anspruch 8, wobei die Detektionsmaßnahme eine Antikörperreaktion umfaßt.

**13.** Verfahren zur Untersuchung eines Menschen, bei dem der Verdacht auf Brustkrebs besteht, auf die Gegenwart eines Retrovirus, wobei man eine Kultur von Monozyten oder mononukleären Zellen, die dem zu untersuchenden Individiuum entnommen worden sind, einer Inkubation in einer Kultur in Gegenwart einer Menge eines Glucocorticoids oder eines Leukämie- oder eines ein anderes Virus induzierenden Agens untergeht, wobei diese Menge, wenn ein Retrovirus vorhanden ist, ausreichend ist, um im Überstand Teilchen zu erzeugen, die das Retrovirus enthalten, und wobei man die Teilchen, wenn sie auftreten, einem Untersuchungsverfahren unterzieht, durch das die Gegenwart des Virus bestimmt wird.

**14.** Verfahren gemäß Anspruch 13, wobei man die Kultur der Monozyten, die dem zu untersuchenden Individiuum entnommen worden sind, zentrifugiert, und wobei man den zentrifugierten Überstand Untersusuchungsmaßnahmen unterzieht, um die Gegenwart des Retrovirus zu bestimmen.

**Revendications**

**1.** Un procédé de formation de fraction contenant un rétrovirus de monocytes ou de cellules mononucléaires humains contenant ledit virus, consistant à préparer une suspension de monocytes ou de cellules mononucléaires isolés d'un être humain présentant, ou, suspecté de souffrir, d'un cancer du sein, dans un milieu de culture, l'incubation de ladite culture et l'isolement du surnageant de ladite culture de la culture incubée, ce procédé étant caractérisé en ce qu'une quantité efficace d'un glucocorticoïde ou d'un agent inducteur viral ou leucémique est ajouté à la suspension avant et/ou durant l'incubation desdites cultures.

**2.** Un procédé selon la revendication 1, comprenant une étape de séparation d'une fraction contenant le rétrovirus du surnageant isolé.

**3.** Un procédé selon la revendication 1, dans lequel l'hormone glucocorticoïde est sous forme de dexaméthasone.

**4.** Un procédé selon la revendication 1, dans lequel l'agent inducteur viral ou leucémique est l'acétate et myristate de phorbol ou la 5' azacytidine.

**5.** Un procédé selon la revendication 4, dans lequel le milieu de culture est formé de milieu de Eagle's enrichi de 10% de sérum de veau foetal, ladite durée d'incubation étant de 3 à 30 jours et la quantité efficace d'acétate myristate de phorbol étant de 330ng par ml de milieu de culture utilisé pour l'incubation.

**6.** Un procédé selon la revendication 5, dans lequel l'incubation est effectuée à une température de l'ordre de 37°C sous une atmosphère d'air contenant 5% de $CO_2$.

**7.** Un procédé pour détecter la présence de rétrovirus dans des monocytes humain ou des cellules mononucléaires d'une personne, souffrant ou suspectée de souffrir d'un cancer du sein, consistant à soumettre une culture desdites cellules à une incubation en présence d'une hormone glucocorticoïde ou d'un dérivé actif en provenant ou d'un agent inducteur viral ou leucémique pour former une fraction contenant un rétrovirus puis à soumettre cette fraction à des protocoles d'essais aux fins de détecter la présence dudit virus.

**8.** Un procédé selon la revendication 7, consistant à incuber lesdites cellules de culture en présence d'acétate et myristate de phorbol (330ng par ml de milieu utilisé pour l'incubation) $10^{-6}$ M de dexaméthasone ou 15 $\mu$M de 5' azacytidine, ladite fraction contenant un rétrovirus étant soumise à des moyens de détection.

**9.** Un procédé selon la revendication 8, dans lequel lesdits moyens de détection consistent en des tests de transcriptase inverse.

**10.** Un procédé selon la revendication 8, dans lequel les moyens de détection consistent en microscopie électronique à coloration négative.

**11.** Un procédé selon la revendication 8, dans lequel les moyens de détection comprennent la formation de cellules géantes.

**12.** Un procédé selon la revendication 8, dans lequel les moyens de détection consistent en une réaction anticorps.

**13.** Un procédé de dépistage systématique sur une population humaine risquant de souffrir du cancer du sein, en vue de déterminer la présence de rétrovirus, consistant à soumettre une culture de cellules mononucléaires ou de monocytes prélevées sur la personne examinée à une incubation dans une culture en présence d'une quantité d'un glucocorticoïde ou d'un agent inducteur viral ou leucémique suffisante pour engendrer, dans le surnageant, en présence de rétrovirus, des particules renfermant ledit rétrovirus et à soumettre lesdites particules, si elles existent, à un protocole d'essai permettant de déterminer la présence desdits virus.

**14.** Un procédé selon la revendication 13 consistant à soumettre ladite culture de monocytes, prélevée sure une personne soumise au dépistage à une centrifugation et à soumettre ce surnageant centrifugé à des moyens de dépistage pour déterminer la présence dudit rétrovirus.